# EUROPÄISCHE PATENTSCHRIFT

(11) **EP 1 125 954 B1**
(45) Veröffentlichungstag und Bekanntmachung des Hinweises auf die Patenterteilung: **15.07.2009**
(21) Anmeldenummer: 01101421.4
(22) Anmeldetag: 23.01.2001
(51) Int. Cl.: C08F 283/06, A61K 47/34, A61K 9/28, A61K 9/20

(54) **Verfahren zur Herstellung und Verwendung von wasserlöslichen oder wasserdispergierbaren polyetherhaltigen Polymerisaten**
Preparation and use of water soluble or water dispersible polyether containing polymers
Préparation et utilisation de polymères solubles ou dispersibles dans l'eau contenant un polyéther

(30) Priorität: 09.02.2000 DE 10005942
(43) Veröffentlichungstag der Anmeldung: 22.08.2001
(73) Patentinhaber: BASF SE, 67056 Ludwigshafen (DE)
(72) Erfinder: Angel, Maximilian, Dr., 67105 Schifferstadt (DE); Gotsche, Michael, Dr., 68167 Mannheim (DE); Kolter, Karl, Dr., 67117 Limburgerhof (DE)

(56) Entgegenhaltungen:
- WO-A-00/18375
- DE-B- 1 077 430

## Beschreibung

Die Erfindung betrifft ein Verfahren zur Herstellung von wasserlöslichen oder wasserdispergierbaren polyetherhaltigen Polymerisaten sowie deren Verwendung als Überzugsmittel, Bindemittel und/oder filmbildende Hilfsstoffe in pharmazeutischen Darreichungsformen oder als Zusatzstoffe in kosmetischen, dermatologischen und/oder hygienischen Zubereitungen, sowie kosmetische, dermatologische, hygienische und/oder pharmazeutische Mittel, enthaltend die erfindungsgemäßen Polymerisate.

Es ist bekannt, Pfropfpolymerisate durch Einwirkung von Ethylenoxid auf Polymere mit aktivem Wasserstoff, wie z.B. Cellulose, Polyamide usw., herzustellen.

In der U.S.-Schrift 2,602,079 wird ein Verfahren zur Herstellung oxalkylierter Polymeren von Vinylestern offenbart. Das dort beschriebene zweistufige Verfahren ist jedoch umständlich und gefährlich, dazu wegen der Verwendung von Lösungsmitteln unwirtschaftlich.

DE 1 077 430, DE 1 094 457 und DE 1 081 229 beschreiben Verfahren zur Herstellung von Pfropfpolymerisaten von Polyvinylestern sowie deren Verwendung als wasserlösliche Verpackungsfolien sowie als Hilfsmittel in der Kosmetik.

WO 00/18375 beschreibt Verfahren zur Herstellung von Pfropfpolymerisaten von Vinylestern, Polyethern und radikalischen Initiatorsystemen, dadurch gekennzeichnet, dass Methanol als Lösungsmittel für das radikalische Initiatorsystem verwendet ist.

In den zitierten Dokumenten wird die Herstellung von Pfropfpolymerisaten aus Polyethylenglykolen und Vinylestern (und weiteren Comonomeren) allgemein beschrieben. Die entsprechenden festen Polyethylenglykole werden dabei in den monomeren Vinylestern gelöst (ggf. unter Zuhilfenahme von Lösungsmitteln), und unter Zusatz von radikalischen Initiatoren polymerisiert. Ein Teil der Mischung wird vorgelegt, anpolymerisiert und der Rest der Mischung zulaufen gelassen.

Bei den so durchgeführten Verfahren bestehen beispielsweise durch das Mischen von Pfropfgrundlage, Monomer und Initiator vor der Polymerisation oder im Zulauf erhebliche Sicherheitsbedenken.

Der vorliegenden Erfindung lag daher die Aufgabe zugrunde, ein sicheres, auch für großtechnische Anwendungen einsetzbares Verfahren mit guter Dosiermöglichkeit des radikalischen Initiators, auch bei geringen Mengen, zu entwickeln, welches die genannten Nachteile nicht aufweist.

Die Aufgabe wurde erfindungsgemäß gelöst, durch ein Verfahren zur Herstellung von Pfropfpolymerisaten von Polyvinylestern durch Polymerisation von
a) mindestens einem Vinylester von aliphatischen C₁-C₂₄-Carbon-säuren, in Gegenwart von
b) bei Raumtemperatur festen Polyethern der allgemeinen Formel I
c) sowie gegebenenfalls mindestens eines weiteren Monomers,
unter Verwendung eines radikalischen Initiatorsystems, dadurch gekennzeichnet, daß ein flüssiges Polyalkylenglykol als Lösungsmittel für das radikalische Initiatorsystem verwendet wird.

Die Polymerisation kann nach den üblichen Polymerisationsverfahren durchgeführt werden. Die Polymerisation kann sowohl im Batch oder vorzugsweise als Zulaufverfahren ausgeführt werden.

Bei der Polymerisation im Batchverfahren kann man so vorgehen, daß man die polyetherhaltige Verbindung b) in mindestens einem Monomer der Gruppe a) und eventuell weiteren Comonomeren der Gruppe c) löst und nach Zugabe eines Polymerisationsinitiators die Mischung auspolymerisiert. Im Zulaufverfahren wird die Polymerisation halbkontinuierlich so durchgeführt, indem man zunächst einen Teil, z.B. 10 % des zu polymerisierenden Gemisches aus der polyetherhaltigen Verbindung b), mindestens einem Monomeren der Gruppe a), eventuell weiteren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), eventuell weiteren Comonomeren der Gruppe c) und Polymerisationsinitiator entweder auf einmal, absatzweise oder vorzugsweise kontinuierlich parallel mit dem Initiator zufügt und polymerisiert.

Im Gegensatz zu den im Stand der Technik beschriebenen Verfahren wird durch die Lösung des Initiators in flüssigem Polyalkylenglykol eine sichere Reaktionsführung gewährleistet. Das erfindungsgemäße Verfahren hat weiterhin den Vorteil, daß die Zuführung weiterer Pfropfgrundlage zu verbesserten Produkteigenschaften führt, da kein weiteres, d.h. chemisch anderes Lösungsmittel benötigt wird.

Verwendung finden die erfindungsgemäß hergestellten Pfropfpolymerisate als Überzugs- und Bindemittel und/oder filmbildende Hilfsstoffe in pharmazeutischen Darreichungsformen sowie in der Kosmetik.

Als Lösungsmittel für den radikalischen Starter werden bei Raumtemperatur flüssige Polyalkylenglykole eingesetzt. Ihr Molekulargewicht beträgt zwischen 88 bis 1000, vorzugsweise 100 bis 600. Besonders bevorzugt werden Polyethylenglykole eingesetzt.

Unter den bei Raumtemperatur festen Polyethern versteht man Polyether der allgemeinen Formel I, 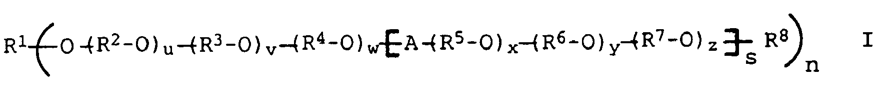 in der die Variablen unabhängig voneinander folgende Bedeutung haben:
- R¹: Wasserstoff, C₁-C₂₄-Alkyl, R⁹-C(=O)-, R⁹-NH-C(=O)-, Polyalkoholrest;
- R⁸: Wasserstoff, C₁-C₂₄-Alkyl, R⁹-C(=O)-, R⁹-NH-C(=O)-;
- R² bis R⁷: -(CH₂)₂-, -(CH₂)₃-, -(CH₂)₄-, -CH₂-CH(CH₃)-, -CH₂-CH(CH₂-CH₃)-, -CH₂-CHOR¹⁰-CH₂-;
- R⁹: C₁-C₂₄-Alkyl;
- R¹⁰: Wasserstoff, C₁-C₂₄-Alkyl, R⁹-C(=0)-;
- A: -C(=O)-O-, -C(=O)-B-C(=O)-O-, -C(=O)-NH-B-NH-C(=O)-O-;
- B: -(CH₂)ₜ-, Arylen, gegebenenfalls substituiert;
- n: 1 bis 8;
- s: 0 bis 500;
- t: 1 bis 12;
- u: 1 bis 5000;
- v: 0 bis 5000;
- w: 0 bis 5000;
- x: 1 bis 5000;
- y: 0 bis 5000;
- z: 0 bis 5000

Bei den erfindungsgemäß hergestellten Pfropfpolymerisaten dienen als Pfropfgrundlage b) generell Polyether der allgemeinen Formel I, ausgewählt aus der Gruppe, bestehend aus Polyalkylenoxiden auf Basis von Ethylenoxid, Propylenoxid und Butylenoxid sowie Polyglycerin. Je nach Art der Monomerbausteine ergeben sich Polymere mit folgenden Struktureinheiten.
-(CH₂)₂-O-, -(CH₂)₃-O-, -(CH₂)₄-O-, -CH₂-CH(CH₃)-O-, -CH₂-CH(CH₂-CH₃)-O-, -CH₂-CHOR⁷-CH₂-O-;

Dabei kann es sich sowohl um Homopolymere als auch um Copolymere handeln, wobei die Copolymere statistisch verteilt sein können oder als sogenannte Blockpolymere vorliegen.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften Polyethern der Formel I und Homo- oder Copolymerisaten der Monomeren a) und gegebenenfalls weiteren Monomeren c) zu verstehen.

Die endständigen primären Hydroxylgruppen der, auf Basis von Alkylenoxiden oder Glycerin hergestellten Polyether sowie außerdem die sekundären OH-Gruppen von Polyglycerin können sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden.

Als Alkylreste für R¹ und R⁸ bis R¹⁰ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der als Pfropfgrundlage verwendeten Polyether liegt im Bereich von 1000 bis 500000, bevorzugt im Bereich von 1000 bis 100000, besonders bevorzugt im Bereich von 1000 bis 20000, ganz besonders bevorzugt im Bereich von 1000 bis 15000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 Mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 Mol.-%, der Propylenoxidanteil 1 bis 60 Mol.-% und der Anteil an Butylenoxid in den Copolymerisaten 1 bis 30 Mol.-%. Neben geradkettigen können auch verzweigte Homo- oder Copolymerisate als Pfropfgrundlage verwendet werden.

Dabei können Polymerisate gebildet werden, bei denen mindestens eine, bevorzugt eine bis acht, besonders bevorzugt eine bis fünf der in den Polyalkoholen vorhandenen Hydroxylgruppen in Form einer Etherbindung mit dem folgenden Polyetherrest P, gemäß Formel I 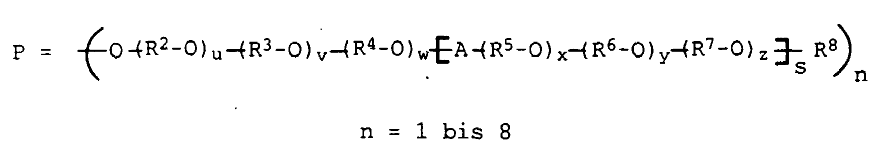 verknüpft sein können.

Die Alkylenoxid-Einheiten können im Polymerisat statistisch verteilt sein oder in Form von Blöcken vorliegen.

Es ist aber auch möglich, Polyester von Polyalkylenoxiden und aliphatischen C₁-C₁₂-, bevorzugt C₁-C₆-Dicarbonsäuren oder aromatischen Dicarbonsäuren, z.B. Oxalsäure, Bernsteinsäure, Adipinsäure oder Terephthalsäure mit Molmassen von 1500 bis 25000, beschrieben in EP-A-0 743 962, als Pfropfgrundlage zu verwenden.

Es ist weiterhin möglich, durch Phosgenierung hergestellte Polycarbonate von Polyalkylenoxiden oder auch Polyurethane von Polyalkylenoxiden und aliphatischen C₁-C₁₂-, bevorzugt C₁-C₆-Diisocyanaten oder aromatischen Diisocyanaten, z.B. Hexamethylendiisocyanat oder Phenylendiisocyanat als Pfropfgrundlage zu verwenden.

Die o.g. Polyester, Polycarbonate oder Polyurethane können bis zu 500, bevorzugt bis zu 100 Polyalkylenoxideinheiten enthalten, wobei die Polyalkylenoxideinheiten sowohl aus Homopolymeren als auch aus Copolymeren unterschiedlicher Alkylenoxide bestehen können.

Bevorzugt werden Polymerisate hergestellt, die dadurch gekennzeichnet sind, daß sie erhältlich sind durch Polymerisation von
a) mindestens einem Vinylester von aliphatischen C₁-C₂₄-Carbon-säuren, in Gegenwart von
b) bei Raumtemperatur festen Polyethern der allgemeinen Formel I,
   in der die Variablen unabhängig voneinander folgende Bedeutung haben:
   - R¹: Wasserstoff, C₁-C₂₄-Alkyl, R⁹-C(=O)-, Polyalkoholrest;
   - R⁸: Wasserstoff, C₁-C₂₄-Alkyl, R⁹-C(=O)-;
   - R² bis R⁷: -(CH₂)₂-, -(CH₂)₃-, -(CH₂)₄-, -CH₂-CH(CH₃)-, -CH₂-CH(CH₂-CH₃)-, -CH₂-CHOR¹⁰-CH₂-;
   - R⁹: C₁-C₂₄-Alkyl;
   - R¹⁰: Wasserstoff, C₁-C₂₄-Alkyl, R⁹-C(=O)-;
   - n: 1 bis 8;
   - s: 0;
   - u: 1 bis 5000;
   - v: 0 bis 5000;
   - w: 0 bis 5000.
c) sowie gegebenenfalls eines weiteren Monomers,
unter Verwendung eines radikalischen Initiatorsystems, dadurch gekennzeichnet, daß ein flüssiges Polyalkylenglykol als Lösungsmittel für das radikalische Initiatorsystem verwendet wird.

Besonders bevorzugt werden Polymerisate hergestellt, die dadurch gekennzeichnet sind, daß sie erhältlich sind durch Polymerisation von
a) mindestens einem Vinylester von aliphatischen C₁-C₁₂-Carbon-säuren, in Gegenwart von
b) bei Raumtemperatur festen Polyethern der allgemeinen Formel I mit einem mittleren Molekulargewicht von größer 1000, in der die Variablen unabhängig voneinander folgende Bedeutung haben:
   - R¹: Wasserstoff, C₁-C₁₂-Alkyl, Polyalkoholrest;
   - R⁸: Wasserstoff, C₁-C₁₂-Alkyl;
   - R² bis R⁷: -(CH₂)₂-, -(CH₂)₃-, -(CH₂)₄-, -CH₂-CH(CH₃)-, -CH₂-CH(CH₂-CH₃)-, -CH₂-CHOR¹⁰-CH₂-;
   - R¹⁰: Wasserstoff, C₁-C₁₂-Alkyl;
   - n: 1 bis 5;
   - s: 0;
   - u: 2 bis 2000;
   - v: 0 bis 2000;
   - w: 0 bis 2000.
c) sowie gegebenenfalls eines weiteren Monomers,
unter Verwendung eines radikalischen Initiatorsystems, dadurch gekennzeichnet, daß ein flüssiges Polyalkylenglykol als Lösungsmittel für das radikalische Initiatorsystem verwendet wird.

Ganz besonders bevorzugt werden Polymerisate hergestellt, dadurch gekennzeichnet, daß
mindestens ein Vinylester von aliphatischen C₁-C₆-Carbonsäuren, insbesondere Vinylacetat, in Gegenwart von
bei Raumtemperatur festen Polyethern der allgemeinen Formel I mit einem mittleren Molekulargewicht von größer 1000, bevorzugt von 1000 bis 15 000, in der die Variablen unabhängig voneinander folgende Bedeutung haben:
- R¹, R⁸: Wasserstoff, C₁-C₆-Alkyl, insbesondere Wasserstoff;
- R² bis R⁷: -(CH₂)₂-, -(CH₂)₃-, -CH₂-CH(CH₃)-, -CH₂-CHOR¹⁰-CH₂-, insbesondere -(CH₂)₂-;
- R¹⁰: Wasserstoff, C₁-C₆-Alkyl;
- n: 1;
- s: 0;
- u: 5 bis 500;
- v: 0 bis 500, insbesondere 0;
- w: 0 bis 500, insbesondere 0
c) sowie gegebenenfalls eines weiteren Monomers umgesetzt wird,
unter Verwendung eines radikalischen Initiatorsystems, dadurch gekennzeichnet, daß ein flüssiges Polyalkylenglykol als Lösungsmittel für das radikalische Initiatorsystem verwendet wird.

Als Polyether können jedoch auch bei Raumtemperatur feste Silikonderivate eingesetzt werden.

Bevorzugte Vertreter solcher polyetherhaltigen Silikonderivaten sind solche, die die folgenden Strukturelemente enthalten: 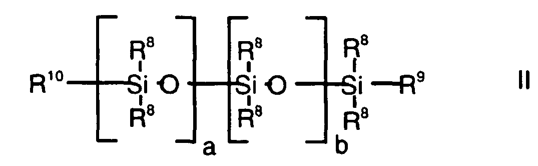 wobei:
R⁹ = CH₃ oder 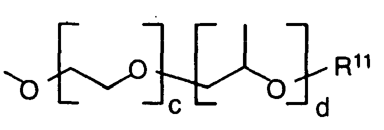 R¹⁰ = CH₃ oder R⁹
R¹¹ = H, CH₃, 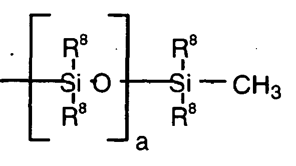 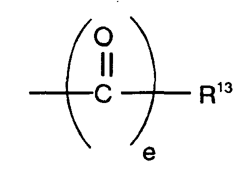 R¹³ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,
und wobei die Reste R⁸ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹² sind, wobei:
R¹² = -(CH₂)_{f} 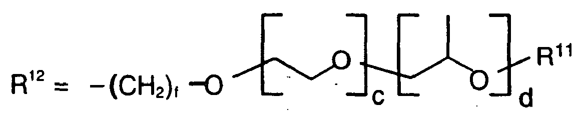 mit der Maßgabe, daß mindestens einer der Reste R⁸, R⁹ oder R¹⁰ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist, und e 0 oder 1 ist.

Bevorzugte Reste R⁹ und R¹² sind solche, bei denen die Summe aus c+d zwischen 5 und 30 beträgt.

Bevorzugt werden die Gruppen R⁸ aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und R¹².

Besonders geeignete Reste R¹¹ sind solche, bei denen im Falle von R¹¹ = -(CO)ₑ-R¹³ R¹³ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹³ sind, für den Fall e=0, Phosphat und Sulfat.

Besonders bevorzugte polyetherhaltige Silikonderivate sind solche der allgemeinen Struktur:

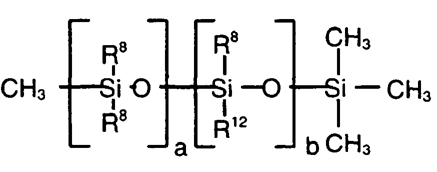

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallyamide oder Polyalkylenoxidvinylamide verwendet werden. Selbstverständlich können auch Copolymerisate solcher Monomere mit anderen ethylenisch ungesättigten Monomeren eingesetzt werden.

Als polyetherhaltige Verbindungen b) können aber auch Umsetzungsprodukte von Polyethyleniminen mit Akylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Für die Polymerisation in Gegenwart der Polyether der Formel I seien als Komponente a) folgende copolymerisierbare Monomere genannt:

Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbon-säuren, insbesondere der C₁-C₆-Carbonsäuren verwendet, ganz besonders bevorzugt Vinylacetat.

Selbstverständlich können auch Mischungen der jeweiligen Monomeren aus der Gruppe a) pfropfpolymerisiert werden.

Die Vinylester (a) können daneben auch in Mischung mit einem oder mehreren, ethylenisch ungesättigten copolymerisierbaren Comonomeren (c) eingesetzt werden, wobei der Anteil dieser zusätzlichen Monomeren auf maximal 50 Gew.-% beschränkt sein sollte. Bevorzugt sind Anteile von 0 bis 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten zusätzlich eingesetzten ethylenisch ungesättigten Comonomere (c) können durch die folgende allgemeine Formel beschrieben werden:

X-C(O)CR¹⁵=CHR¹⁴

wobei
X ausgewählt ist aus der Gruppe der Reste -OH, -OM, -OR¹⁶, NH₂, -NHR¹⁶, N(R¹⁶)₂;
M ist ein Kation ausgewählt aus der Gruppe bestehend aus: Na⁺, K⁺, Mg⁺⁺, Ca⁺⁺, Zn⁺⁺, NH₄⁺, Alkylammonium, Dialkylammonium, Trialkylammonium und Tetraalkylammonium;
die Reste R¹⁶ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.
R¹⁵ und R¹⁴ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III) 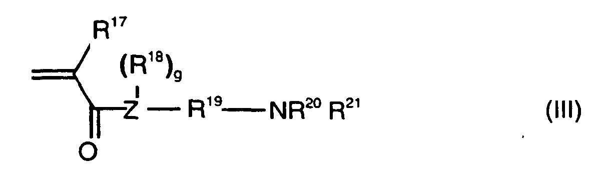 mit
- R¹⁷ =: H, Alkyl mit 1 bis 8 C-Atomen,
- R¹⁸ =: H, Methyl,
- R¹⁹ =: Alkylen mit 1 bis 24 C-Atomen, optional substituiert durch Alkyl,
- R²⁰, R²¹ =: C₁-C₄₀ Alkylrest,
- Z =: Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl-(meth)acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyl- oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin R²² bis R²⁴ unabhängig voneinander für Wasserstoff, C₁-C₄-Alkyl oder Phenyl steht:

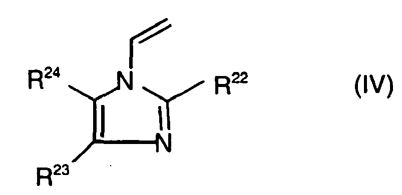

Weitere geeignete Comonomere (c) sind Diallylamine der allgemeinen Formel (V) 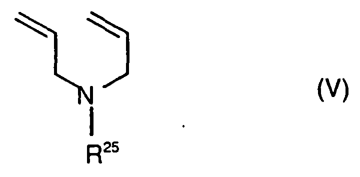 mit R²⁵ = C₁- bis C₂₄-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)-acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)-acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)-butyl]methacrylamid, N-[8-(dimethylamino)octyl]methacrylamid, N-[12-(dimethylamino)dodecyl]methacrylamid, N-[3-(diethylamino)-propyl]methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)-propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden (R²⁶ = C₁- bis C₄₀-Alkyl).

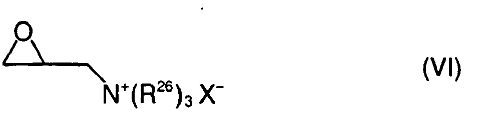

Beispiele hierfür sind zum Beispiel:
(Meth)acryloyloxyhydroxypropyltrimethylammoniumchlorid und
(Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.

Bevorzugt werden silikonfreie Regler eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen, wie zum Beispiel 1,2-Diaminoethan, 1,3-Diaminopropan. Ferner sind Triallylamin oder entsprechende Ammoniumsalze, N-Vinylverbindungen von Harnstoffderivaten, mindestens zweiwertigen Amiden, Cyanuraten oder Urethanen. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Weiterhin eignen sich anorganische Verbindungen wie Borsäure oder Borsäuresalze, beispielsweise Natriummetaborat, Borax (Dinatriumtetraborat), sowie Salze mehrwertiger Kationen, z.B. Kupfer(II)-salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen. Der Anteil Borsäure bzw. Borsäuresalze, bezogen auf die erfindungsgemäßen Polymere, beträgt 0 bis 15 Gew.-%, bevorzugt 0 bis 10 Gew.-%, besonders bevorzugt 0 bis 5 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold^{™}, Ultrahold^{™}, Ultrahold Strong^{™}, Luviflex^{™} VBM, Luvimer^{™}, Acronal^{™}, Acudyne^{™}, Stepanhold^{™}, Lovocryl^{™}, Versatyl^{™}, Amphomer^{™} oder Eastma AQ^{™}.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Die K-Werte der Polymerisate sollen im Bereich von 10 bis 300, bevorzugt 15 bis 150, besonders bevorzugt 15 bis 100, ganz besonders bevorzugt im Bereich von 20 und 80 liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64 und 71 bis 74 (1932) in N-Methylpyrrolidon oder anderen Lösungsmitteln bei 25°C und Polymerkonzentrationen, die je nach K-Wert-Bereich zwischen 0,1 Gew.-% und 5 Gew.-% liegen. Bei vernetzten - z.B. unlöslichen - Polymerisaten kann die Angabe eines K-Werts nicht sinnvoll sein.

Das Mengenverhältnis der als Pfropfgrundlage verwendeten Polyether zu den eingesetzten Vinylestern liegt im Bereich von 1 : 0,5 bis 1 : 50, bevorzugt im Bereich von 1 : 1,5 bis 1 : 35, besonders bevorzugt im Bereich von 1 : 2 bis 1 : 30.

Als Polymerisationsinitiatoren eignen sich bevorzugt organische Peroxide, wie Diacetylperoxid, Dibenzoylperoxid, Succinylperoxid, Di-tert.-butylperoxid, tert.-Butylperbenzoat, tert.-Butylperpivalat, tert.-Butylperoktoat, tert.-Butylpermaleinat, Cumolhydroperoxid, Diisopropylperoxidicarbamat, Bis-(o-toluyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid sowie Mischungen der genannten Initiatoren, Redoxinitiatoren und Azostarter (z.B. Azo-bisisobutyronitril, Azo-bis-(2-amidopropan)dihydrochlorid oder 2-2'-Azo-bis-(2-methyl-butyronitril).

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 10 bar ablaufen.

Zur Erhöhung der Hydrophilie der erfindungsgemäß verwendeten Polymeren können die Estergruppen nach der Polymerisation verseift bzw. teilweise verseift werden. Die Verseifung erfolgt in an sich bekannter Weise durch Zugabe einer Base, bevorzugt durch Zugabe einer methanolischen Natrium- oder Kaliumhydroxidlösung bei Temperaturen im Bereich von 10 bis 80°C, bevorzugt im Bereich von 20 bis 70°C. Der Verseifungsgrad hängt ab von der Menge der eingesetzten Base, von der Verseifungstemperatur und der Verseifungszeit.

Der Verseifungsgrad der Polyvinylestergruppen kann also im Bereich von 0 bis 100 % liegen. Bevorzugt liegt er im Bereich von 20 bis 100 %, besonders bevorzugt im Bereich von 40 bis 100 %, insbesondere von 65 bis 100 % und ganz besonders bevorzugt im 'Bereich von 80 bis 100 %.

Der Feststoffgehalt der erhaltenen wäßrigen Polymerisat-Dispersionen bzw. Lösungen beträgt in der Regel 10 bis 70 Gew.-%, bevorzugt 15 bis 65 Gew.-%, besonders bevorzugt 20 bis 60 Gew.-%.

Je nach Verseifungsgrad und Konzentration erhält man wäßrige Dispersionen oder Lösungen der erfindungsgemäß verwendeten Polymerisate.

Die Polymerisat-Dispersionen oder Lösungen können durch verschiedene Trocknungsverfahren wie z.B. Sprühtrocknung, Fluidized Spray Drying, Walzentrocknung oder Gefriertrocknung in Pulverform überführt werden. Aus dem so erhaltenen Polymer-Trockenpulver läßt sich durch Redispergieren in Wasser erneut eine wäßrige Dispersion bzw. Lösung herstellen läßt. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren Polyalkylenoxid- bzw. Polyglycerin-haltigen Polymerisate eignen sich hervorragend als magensaftlösliche oder als im Magensaft dispergierbare Filmbildner, Bindemittel, Benetzungshilfsstoff und/oder Löslichkeitsverbesserer für pharmazeutische Darreichungsformen sowie als Zusatzstoffe für kosmetische, dermatologische oder hygienische Zubereitungen.

Ein weiterer Gegenstand der Erfindung sind kosmetische, dermatologische, hygienische und oder pharmazeutische Mittel, enthaltend die erfindungsgemäßen Polymerisate.

Die nachfolgenden Beispiele sollen das erfindungsgemäße Herstellverfahren näher erläutern:

### Beispiel 1

| | |
|---|---|
| Vorlage | 500 g Pluriol E 6000 |
| Zulauf 1 | 900 g Vinylacetat |
| Zulauf 2 | 8,5 g tertiär-Butylperoktoat |
| | 100 g Pluriol E 600 |

### Apparatur

6-1-JUVO-Kessel mit Ankerrührer, 2 geregelten Zulaufgefäßen, Stickstoffüberleitung, 1 Innenthermometer (unten), Außenthermometer (Badtemperaturmessung) und Temperaturregelung über Prozeßleitsystem

### Fahrweise

Vorlage dreimal mit 9 bar Stickstoff abpressen. Vorlage unter 1 bar Stickstoff bei 60 upm rühren und auf 88°C Innentemperatur aufheizen. Nach Erreichen von 88°C Vorlagentemperatur werden die Zuläufe 1 und 2 in jeweils 5 h zugegeben. Nach Zulaufende wird 3 h bei 88°C nachpolymerisiert.

Anschließend werden ca. 2500 g VE - Wasser langsam zugegeben. Im weiteren Verlauf des Versuchs werden ca. 400 ml des Kesselinhalts, im wesentlichen Wasser und ggf. monomeres Vinylacetat, unter intensiver Stickstoffeinleitung ("Strippen") entfernt.

| | |
|---|---|
| Feststoffgehalt | 41,6 % |
| K-Wert | 17,8 (gemessen als 1 %ige Lösung in VE-Wasser) |

### Beispiel 2

| | |
|---|---|
| Vorlage | 51,0 g Pluriol E 6000 |
| | |
| Zulauf 1 | 340,0 g Vinylacetat |
| | |
| Zulauf 2 | 1,36 g tertiär-Butylperoktoat |
| | |
| | 9,0 g Pluriol E 600 |
| Zulauf 3 | 400 g Methanol |
| | |
| Zulauf 4 | 4,08 g Natriumhydroxid |
| | 36,72 g Methanol |
| | |
| Zulauf 5 | 6,66 g Zitronensäure |
| | 659,2 g VE-Wasser |

Feststoffgehalt 19,8 %
K-Wert 71,1 (gemessen als 1 %ige Lösung in n-Methylpyrrolidon)

### Apparatur

2-1-Pilotrührwerk mit Ankerrührer, Rückflußkühler, 2 geregelten Zulaufgefäßen, Stickstoffüberleitung, 2 Innenthermometer (oben und unten), Außenthermometer und Temperaturregelung über Prozeßleitsystem

### Fahrweise

Vorlage unter leichtem Stickstoffstrom bei 150 upm rühren und auf 90°C Außentemperatur aufheizen. Nachdem das Polyethylenglykol vollständig geschmolzen ist, werden jeweils 10 Gew.-% der Zuläufe 1 und 2 zugegeben und der Versuch 30 Minuten anpolymerisiert. Danach werden der Rest der Zuläufe 1 und 2 in jeweils 3,5 h zugegeben. Nach Zulaufende wird 3 h nachpolymerisiert.

Anschließend wird auf 60°C abgekühlt und der Zulauf 3 unter Rühren zugegeben. Nach dem Abkühlen des Versuchs auf unter 30°C wird der Zulauf 4 unter Rühren zugegeben. Nach ca. 3 bis 10 Minuten ist die Verseifung soweit fortgeschritten, daß das Produkt fest wird (gegebenenfalls Rührer ausschalten). Ca. 30 Minuten nach der Zugabe von Zulauf 4 wird der Zulauf 5 zugegeben und der Versuch auf 85°C Außentemperatur aufgeheizt. Während dem Aufheizen wird der Rührer wieder eingeschaltet. Nachdem das Produkt vollständig gelöst ist, wird das Methanol (und das entstandene Methylacetat) mittels Wasserdampfdestillation abdestilliert.

| | |
|---|---|
| Pluriol E 6000 | Polyethylenglykol mit (mittlerem) Molekulargewicht 6000, in Schuppenform |
| Pluriol E 600 | Polyethylenglykol mit (mittlerem) Molekulargewicht 600, flüssig bei Raumtemperatur |

### Beispiel 3

### Herstellung von Propranolol-HCl Filmtabletten (magensaftlöslicher Überzug)

Auf 9 mm gewölbte Tablettenkerne mit 40 mg Propranolol-HCl (Fa. Knoll AG), 195,0 mg Ludipress® (Fa. BASF Aktiengesellschaft), 12,50 mg Kollidon® VA 64 (Fa. BASF AG) und 2,50 mg Magnesiumstearat wurde in einem Horizontaltrommelcoater (Accela-Cota 24", Fa. Manesty) ein Filmüberzug gemäß folgender Zusammensetzung aufgesprüht:

| | |
|---|---|
| Pfropfpolymer PEG 6000/VAc aus Beispiel 2 | 10,0 Gew.-% |
| Sicovit® rot (Fa. BASF Aktiengesellschaft) | 1,5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 81,0 Gew.-% |

Zur Herstellung der Sprühdispersion wurde das Pfropfpolymerisat in Wasser gelöst, mit Sicovit® rot, Titandioxid und Talkum versetzt und anschließend in einer Korundscheibenmühle homogenisiert. 1260 g (incl. eines Zuschlages von 10 % für Sprühverluste) wurden bei einer Zulufttemperatur von 60°C und einer Sprührate von 30 g/min mit einer 1,0 mm weiten Sprühdüse und einem Sprühdruck von 1,5 bar auf 5 000 g Kerne appliziert. Nach der Sprühung wurde noch 5 min. bei 60°C nachgetrocknet.

Man erhielt sehr glatte, glänzende, rote Filmtabletten mit folgenden Eigenschaften:

| | |
|---|---|
| Aussehen | sehr glatte Oberfläche, schön ausgebildete Gravur |
| Zerfall (künstl. Magensaft) | 5 min. 11 s. |
| Zerfallszeitdifferenz (Filmtablette-Kern) | 42 s. |
| Bruchfestigkeit | 94 N |
| Bruchfestigkeitsdifferenz (Filmtablette-Kern) | 25 N |

### Vergleichsbeispiel

Analog Beispiel 7 wurde anstelle des Pfropfpolymerisats Pharmacoat® 606 (Hydroxypropylmethylcellulose, Fa. Shin-etsu) eingesetzt und wie laut Herstellerangaben empfohlen, 1,0 Gew.-% Polyethylenglykol 6000 (Lutrol® 6000, BASF Aktiengesellchaft) zugesetzt.

Folgende Tabletteneigenschaften wurden erzielt:

| | |
|---|---|
| Aussehe | leicht rauhe Oberfläche, zugeschmierte Gravur |
| Zerfall (künstl. Magensaft) | 11 min. 12 s. |
| Zerfallszeitdifferenz (Filmtablette-Kern) | 6 min. 43 s. |
| Bruchfestigkeit | 87 N |
| Bruchfestigkeitsdifferenz (Filmtablette-Kern) | 18 N |

### Beispiel 4

### Verwendung als Bindemittel in Glibenclamid-Tabletten

890 g Calciumhydrogenphosphat (Fa. Rhone Poulenc) und 30 g Glibenclamid (Fa. Arzneimittelwerk Dresden) wurden über ein 0,8 mm-Sieb gesiebt und in einem Turbulamischer (Fa. Bachofen) 5 min. gemischt. Diese Pulvermischung wurde in einem Stephanmischer (Fa. Stephan) mit 119 g einer 25 gew.-%igen wäßrigen Zubereitung eines Pfropfpolymers aus PEG 6000/VAc (hergestellt wie in Beispiel 2) unter Rühren langsam befeuchtet. Zur vollständigen Durchfeuchtung wurde nach der Zugabe der Bindemittelzubereitung noch 2 min. bei 800 U/min weitergerührt. Die feuchte Masse wurde anschließend durch ein 0,8 mm-Sieb gegeben und auf einer Horde 20 Std. bei 25°C abgetrocknet. Nach der Zugabe von 45 g Kollidon® CL (Fa. BASF) und 5 g Magnesiumstearat (Fa. Bärlocher) erfolgte die Endmischung wiederum im Turbulamischer über 5 min. Diese Tablettiermischung wurde anschließend auf einer Korsch PH 106 Rundläuferpresse (Fa. Korsch) bei 10 kN und 18 kN Preßkraft zu biplanaren, facettierten Tabletten mit 12 mm Durchmesser und 500 mg Gesamtgewicht verpreßt.

| | | |
|---|---|---|
| Eigenschaften | 10 KN Preßkraft | 18 KN Preßkraft |
| | | |
| Bruchfestigkeit | 29 N | 55 N |
| Friabilität | 0,6 % | 0 % |
| Zerfall | 31 s. | 41 s. |

### Vergleichsbeispiel

Die Herstellung erfolgte analog Beispiel 4, jedoch mit Hydroxypropylmethylcellulose (Pharmacoat® 603, Fa. Shin-etsu) als Bindemittel, wobei die Konzentration des Bindemittels in der Lösung aus Viskositätsgründen auf 20 Gew.-% reduziert werden mußte.

| | | |
|---|---|---|
| Eigenschaften | 10 KN Preßkraft | 18 KN Preßkraft |
| | | |
| Bruchfestigkeit | 16 N | 40 N |
| Friabilität | 8,0 % | 0,6 % |
| Zerfall | 35 s. | 58 s. |

### Beispiel 5

### Verwendung als Hilfsstoff zur Herstellung von Ultraschallgelen

5 g p-Hydroxybenzoesäuremethylester wurden bei 50°C in 724 g demineralisiertem Wasser gelöst. Anschließend wurden 6 g Polyacrylsäure (Carbopol® 940, Fa. Goodrich) und 15 g eines Pfropfpolymerisats aus PEG 6000/VAc (Beispiel 2) unter Rühren eingearbeitet. Nach Zugabe von 200 g demineralisiertem Wasser und 50 g 4%iger wäßriger Natronlauge wurde noch 15 min gerührt, wobei darauf geachtet wurde, daß keine Luft eingearbeitet wurde. Es entstand ein Gel mit einem sehr angenehmen Hautgefühl und guten Kontakteigenschaften.

### Beispiel 6

### Verwendung als Filmbildner in einem Desinfektionsspray

150 g eines Pfropfpolymers aus PEG 6000/VAc (Beispiel 2) wurden in 375 demineralisiertem Wasser gelöst und mit 375 g Ethanol versetzt. Anschließend wurden in dieser Polymerlösung unter Rühren 100 g Polyvinylpyrrolidon-Iod (PVP-Jod 30/06, BASF Aktiengesellschaft) gelöst und die Zubereitung in Pumpsprayflaschen abgefüllt. Das Desinfektionsspray zeigte sehr gute Filmeigenschaften auf der Haut und wies keinen Jodverlust nach Lagerung unter Stressbedingungen (7 Tage bei 52°C) auf.

## Patentansprüche

1. Verfahren zur Herstellung von Pfropfpolymerisaten von Polyvinylestern durch Polymerisation von
a) mindestens einem Vinylester von aliphatischen C₁-C₂₄-Carbonsäuren, in Gegenwart von
b) bei Raumtemperatur festen Polyethern der allgemeinen Formel I 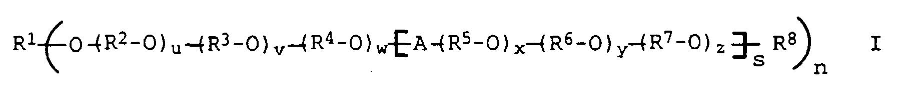 in der die Variablen unabhängig voneinander folgende Bedeutung haben:
R¹ Wasserstoff, C₁-C₂₄-Alkyl, R⁹-C(=O)-, R⁹-NH-C(=O)-, Polyalkoholrest;
R⁸ Wasserstoff, C₁-C₂₄-Alkyl, R⁹-C(=O)-, R⁹-NH-C(=O)-;
R² bis R⁷ -(CH₂)₂-, -(CH₂)₃-, -(CH₂)₄-, -CH₂-CH(CH₃)-, -CH₂-CH(CH₂-CH₃)-, -CH₂-CHOR¹⁰-CH₂-;
R⁹ C₁-C₂₄-Alkyl;
R¹⁰ Wasserstoff, C₁-C₂₄-Alkyl, R⁹-C(=O)-_{;}
A -C(=O)-O-, -C(=O)-B-C(=O)-O-, -C(=O)-NH-B-NH-C(=O)-O-;
B -(CH₂)ₜ-, Arylen, gegebenenfalls substituiert;
n 1 bis 8;
s 0 bis 500;
t 1 bis 12;
u 1 bis 5000;
v 0 bis 5000;
w 0 bis 5000;
x 1 bis 5000;
y 0 bis 5000;
z 0 bis 5000
c) sowie gegebenenfalls mindestens eines weiteren Monomers
unter Verwendung eines radikalischen Initiatorsystems, **dadurch gekennzeichnet, daß** flüssiges Polyalkylenglykol als Lösungsmittel für das radikalische Initiatorsystem verwendet wird.

2. Verfahren gemäß Anspruch 1, **dadurch gekennzeichnet, daß** die Zugabe der Lösung des radikalischen Initiatorsystems kontinuierlich über die gesamte Reaktionszeit der Polymerisation erfolgt.

3. Verfahren nach einem der Ansprüche 1 bis 2, **dadurch gekennzeichnet, daß** als Lösungsmittel für den radikalischen Starter bei Raumtemperatur flüssiges Polyethylenglykol verwendet wird.

4. Verwendung der nach einem Verfahren gemäß einem der Ansprüche 1 bis 3 hergestellten Polymerisate als Überzugs-, Bindemittel und/oder filmbildende Hilfsstoffe für pharmazeutische Darreichungsformen.

5. Verwendung der nach einem Verfahren gemäß einem der Ansprüche 1 bis 3 hergestellten Polymerisate als Zusatzstoffe für kosmetische, hygienische und/oder dermatologische Zubereitungen.

6. Kosmetische, dermatologische, hygienische und oder pharmazeutische Darreichungsformen enthaltend mindestens eines, der nach einem Verfahren der Ansprüche 1 bis 3 hergestellten Polymerisate neben üblichen Hilfsstoffen.

7. Pfropfpolymerisate von Polyvinylestern erhältlich durch Polymerisation von
a) mindestens einem Vinylester von aliphatischen C₁-C₂₄-Carbonsäuren, in Gegenwart von
b) bei Raumtemperatur festen Polyethern der allgemeinen Formel I 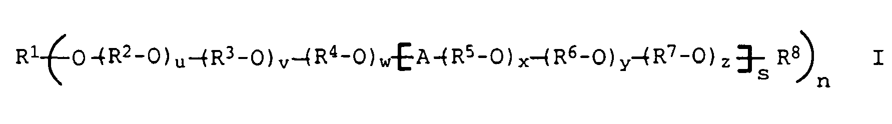 in der die Variablen unabhängig voneinander folgende Bedeutung haben:
R¹ Wasserstoff, C₁-C₂₄-Alkyl, R⁹-C(=O)-, R⁹-NH-C(=O)-, Polyalkoholrest;
R⁸ Wasserstoff, C₁-C₂₄-Alkyl, R⁹-C(=O)-, R⁹-NH-C(=O)-_{;}
R² bis R⁷ -(-CH₂)₂-, -(CH₂)₃-, -(CH₂)₄-, -CH₂-CH(CH₃)-, -CH₂-CH(CH₂-CH₃)-, -CH₂-CHOR¹⁰-CH₂-;
R⁹ C₁-C₂₄-Alkyl;
R¹⁰ Wasserstoff, C1-C₂₄-Alkyl, R⁹-C(=O)-;
A -C(=O)-O-, -C(=O)-B-C(=O)-O-, -C(=O)-NH-B-NH-C(=O)-O-;
B -(CH₂)ₜ-, Arylen, gegebenenfalls substituiert;
n 1 bis 8;
s 0 bis 500;
t 1 bis 12;
u 1 bis 5000;
v 0 bis 5000;
w 0 bis 5000;
x 1 bis 5000;
y 0 bis 5000;
z 0 bis 5000
c) sowie gegebenenfalls mindestens eines weiteren Monomers
unter Verwendung eines radikalischen Initiatorsystems, **dadurch gekennzeichnet, daß** flüssiges Polyalkylenglykol als Lösungsmittel für das radikalische Initiatorsystem verwendet wird.

## Claims

1. A process for preparing graft copolymers of polyvinyl esters by polymerization of
a) at least one vinyl ester of aliphatic C₁-C₂₄-carboxylic acids in the presence of
b) polyethers which are solid at room temperature and have the general formula I 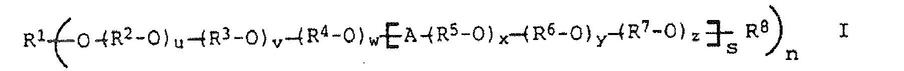 in which the variables have the following meaning, independently of one another:
R₁ hydrogen, C₁-C₂₄-alkyl, R⁹-C(=O)-, R⁹-NH-C(=O)-, polyalcohol residue;
R⁸ hydrogen, C₁-C₂₄-alkyl, R⁹-C(=O)-, R⁹-NH-C(=O)-;
R² to R⁷ (-CH₂)₂-, - (CH₂)₃-_{,} - (CH₂)₄-, -CH₂-CH(CH₃)-,-CH₂-CH(CH₂-CH₃) -, -CH₂-CHOR¹⁰-CH₂;
R⁹ C₁-C₂₄-alkyl;
R¹⁰ hydrogen, C₁-C₂₄-alkyl, R⁹-C(=O)-;
A -C(=O)-O-, -C(=O)-B-C(=O)-O-, -C(=O)-NH-B-NH-C(=O)-O-;
B -(CH₂)ₜ-, arylene, optionally substituted;
n 1 to 8;
s 0 to 500;
t 1 to 12;
u 1 to 5000;
v 0 to 5000;
w 0 to 5000;
x 1 to 5000;
y 0 to 5000;
z 0 to 5000
c) and, where appropriate, at least one other monomer
using a free-radical initiator system, wherein liquid polyalkylene glycol is used as solvent for the free-radical initiator system.

2. The process according to claim 1, wherein the solution of the free-radical initiator system is added continuously throughout the polymerization reaction time.

3. The process according to either of claims 1 and 2, wherein liquid polyethylene glycol is used as solvent for the free-radical initiator at room temperature.

4. The use of the polymers prepared by a process according to any of claims 1 to 3 as coating agents, binders and/or film-forming excipients for pharmaceutical dosage forms.

5. The use of the polymers prepared by a process according to any of claims 1 to 3 as additives to cosmetic, hygienic and/or dermatological preparations.

6. A cosmetic, dermatological, hygienic or pharmaceutial dosage form comprising at least one of the polymers prepared by a process according to claims 1 to 3 in addition to conventional excipients.

7. Graft copolymers of polyvinyl esters obtainable by polymerization of
a) at least one vinyl ester of aliphatic C₁-C₂₄-carboxylic acids in the presence of
b) polyethers which are solid at room temperature and have the general formula I 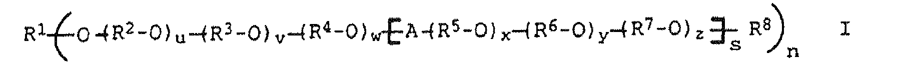 in which the variables have the following meaning, independently of one another:
R¹ hydrogen, C₁-C₂₄-alkyl, R⁹-C(=O)-, R⁹-NH-C (=O) -, polyalcohol residue;
R⁸ hydrogen, C₁-C₂₄-alkyl, R⁹-C(=O)-, R⁹-NH-C(=O)-;
R² to R⁷ -(CH₂)₂-, -(CH₂)₃-, -(CH₂)₄-, -CH₂-CH(CH₃)-,-CH₂-CH(CH₂-CH₃)-, -CH₂-CHOR¹⁰-CH₂- ;
R⁹ C₁-C₂₄-alkyl;
R¹⁰ hydrogen, C₁-C₂₄-alkyl, R⁹-C (=O)-;
A -C (=O) -O-, -C(=O)-B-C(=O)-O-, -C(=O)-NH-B-NH-C(=O)-O-;
B -(CH₂)ₜ-, arylene, optionally substituted;
n 1 to 8;
s 0 to 500;
t 1 to 12;
u 1 to 5000;
v 0 to 5000;
w 0 to 5000;
x 1 to 5000;
y 0 to 5000;
z 0 to 5000
c) and, where appropriate, at least one other monomer
using a free-radical initiator system, wherein liquid polyalkylene glycol is used as solvent for the free-radical initiator system.

## Revendications

1. Procédé pour la préparation de polymères greffés de poly(esters vinyliques) par polymérisation de
a) au moins un ester vinylique d'acides carboxyliques aliphatiques en C₁-C₂₄, en présence de
b) polyéthers solides à la température ambiante, de formule générale I 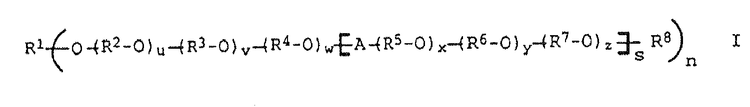 dans laquelle les variables ont, indépendamment les unes des autres, les significations suivantes :
R¹ représente un atome d'hydrogène, un groupe alkyle en C₁-C₂₄, R⁹-C(=O)-, R⁹-NH-C(=O)-, un reste de polyalcool ;
R⁸ représente un atome d'hydrogène, un groupe alkyle enC₁₋C₂₄R⁹-C(=O)-, R⁹-NH-C(=O-;
R² à R⁷ représentent
-(CH₂)₂-, -(CH₂)₃-, -(CH₂)₄-, -CH₂-CH (CH₃)-, -CH₂-CH ( CH₂-CH₃ ) -, -CH₂-CHOR¹⁰-CH₂- ;
R⁹ représente un groupe alkyle en C₁-C₂₄ ;
R¹⁰ représente un atome d'hydrogène, un groupe alkyle en C₁C₂₄, R⁹-C (=O) - ;
A représente -C(=O)-O-, -C(=O-B-C(=O-O, -C(=O-NH-B-NH-C(=O-O ;
B représente un groupe -(CH₂)ₜ-, arylène, éventuellement substitué ;
n va de 1 à 8 ;
s va de 0 à 500 ;
t va de 1 à 12 ;
u va de 1 à 5 000 ;
v va de 0 à 5 000 ;
w va de 0 à 5 000 ;
x va de 1 à 5 000 ;
y va de 0 à 5 000 ;
z va de 0 à 5 000
c) ainsi qu'éventuellement d'au moins un autre monomère,
avec utilisation d'un système amorceur radicalaire, **caractérisé en ce que** pour le système amorceur radicalaire on utilise un polyalkylèneglycol liquide en tant que solvant.

2. Procédé selon la revendication 1, **caractérisé en ce que** l'addition de la solution du système amorceur radicalaire s'effectue en continu pendant toute la durée de la polymérisation.

3. Procédé selon la revendication 1 ou 2, **caractérisé en ce que** pour l'amorceur radicalaire on utilise comme solvant un polyéthylèneglycol liquide à la température ambiante.

4. Utilisation des polymères préparés conformément à un procédé selon l'une quelconque des revendications 1 à 3, en tant que liants de revêtement et/ou adjuvants filmogènes pour des formes d'administration pharmaceutiques.

5. Utilisation des polymères préparés conformément à un procédé selon l'une quelconque des revendications 1 à 3, en tant qu'additifs pour des préparations cosmétiques, d'hygiène et/ou dermatologiques.

6. Formes d'administration cosmétiques, dermatologiques, d'hygiène et/ou pharmaceutiques contenant au moins l'un des polymères préparés selon un procédé des revendications 1 à 3, en plus d'adjuvants usuels.

7. Polymères greffés de poly(esters vinyliques) pouvant être obtenus par polymérisation de
a) au moins un ester vinylique d'acides carboxyliques aliphatiques en C₁-C₂₄ en présence de
b) polyéthers solides à la température ambiante, de formule générale I 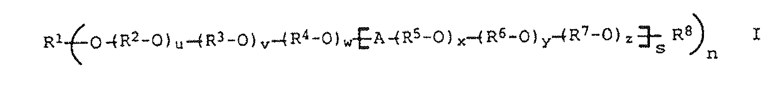 dans laquelle les variables ont, indépendamment les unes des autres, les significations suivantes :
R¹ représente un atome d'hydrogène, un groupe alkyle en C₁-C₂₄, R⁹-C (=O -, R⁹-NH-C (=O -, un reste de polyalcool ;
R⁸ représente un atome d'hydrogène, un groupe alkyle en C₁-C₂₄, R⁹-C(=O-, R⁹-NH-C(=O)-;
R² à R⁷ représentent
-(CH₂)₂-, (CH₂)₃-, (CH₂)₄-,-CH₂-CH(CH₃)-, -CH₂-CH(CH₂-CH₃)-, -CH₂-CHOR¹⁰-CH₂- ;
R⁹ représente un groupe alkyle en C₁-C₂₄ ;
R¹⁰ représente un atome d'hydrogène, un groupe alkyle en C₁-C₂₄, R⁹-C(=O)- ;
A représente -C(=O)-O-, -C(=O)-B-C(=O)-, -C(=O)-NH-B-NH-C(=O)-O- ;
B représente un groupe -(CH₂)ₜ-, arylène, éventuellement substitué ;
n va de 1 à 8 ;
s va de 0 à 500 ;
t va de 1 à 12 ;
u va de 1 à 5 000 ;
v va de 0 à 5 000 ;
w va de 0 à 5 000 ;
x va de 1 à 5 000 ;
y va de 0 à 5 000 ;
z va de 0 à 5 000
c) ainsi qu'éventuellement d'au moins un autre monomère,
avec utilisation d'un système amorceur radicalaire, **caractérisé en ce que** pour le système amorceur radicalaire on utilise un polyalkylèneglycol liquide en tant que solvant.
